# EUROPEAN PATENT APPLICATION

(11) **EP 2 098 500 A1**
(43) Date of publication of application: **09.09.2009**
(21) Application number: 08384002.5
(22) Date of filing: 07.03.2008
(51) Int. Cl.: C07C 57/40, C07C 63/70, C07C 65/01, C07C 211/38, A61K 31/13, A61K 31/192, A61P 25/02, A61P 29/00, A61P 25/28

(54) **Salts of menantine and cox-inhibitors and their crystal form in the treatment of pain**

(71) Applicant: Laboratorios Del. Dr. Esteve, S.A., 08041 Barcelona (ES)
(72) Inventor: Buschmann, Helmut, Heinrich, Dr., 52076 Aachen (Walheim) (DE); Tesson, Nicolas, 08028 Barcelona (ES); Farran, Joan, 08028 Barcelona (ES); Rafecas, Llorenc, 08028 Barcelona (ES)
(74) Representative: Peters, Hajo

(57) **Abstract**

The present invention relates to salts of Memantine and COX-INHIBITORs, their crystal form, the processes for preparation of the same and their uses as medicaments, more particularly for the treatment of pain.

## Description

The present invention relates to salts of Memantine and COX-INHIBITORs, their crystal from, and their specific polymorphs, the processes for preparation of the same and their uses as medicaments, more particularly for the treatment of pain.

Pain is a complex response that has been functionally categorized into sensory, autonomic, motor, and affective components. The sensory aspect includes information about stimulus location and intensity while the adaptive component may be considered to be the activation of endogenous pain modulation and motor planning for escape responses. The affective component appears to include evaluation of pain unpleasantness and stimulus threat as well as negative emotions triggered by memory and context of the painful stimulus.

In general, pain conditions can be divided into chronic and acute. Chronic pain includes neuropathic pain and chronic inflammatory pain, for example arthritis, or pain of unknown origin, as fibromyalgia. Acute pain usually follows non-neural tissue injury, for example tissue damage from surgery or inflammation, or migraine.

One compound whose main focus currently is not in pain, but which nevertheless did show initial success in diabetic neuropathy and also chronic and arthritic pain in animal models is the NMDA receptor antagonist Memantine. Besides these animal models a number of clinical trials were undertaken, which resulted also in proving the efficacy of Memantine in pain, but at least in one case did not reach the endpoint which was envisioned. Therefore, even though there is no doubt that memantine has potential and efficacy in the treatment of pain, the current free base or hydrochlorid salt used in the trials under certain less than optimal conditions did not seem to be sufficient for a clinical success. Therefore, there is a clear need for alternatives, especially new salts of memantine, that would enhance the usuablility or efficacy of memantine in pain, especially in neuropathic pain and under clinical conditions.

Memantine is currently marketed for the treatment of Alzheiner's Disease. Memantine (1-amino-3,5-dimethyl-adamantane). Memantine - whose empirical formula as a free base is C₁₂H₂₁N - has a pKa of 10. 7. Memantine free base has the following formula:

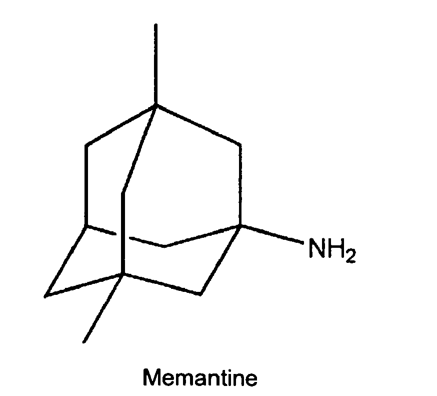

Memantine is available as a free base but also is available or described in form of a number of salts, including salts with HCl, HBr, HI, butenedioic acid, as nitrate, sulfate, phosphate, oxalate, citrate, methanesulfonate, toluenesulfonate, tartrate 1,6-hexandioate, 3-amino-propanesulfonate, N-Vinylsuccinamic acid, or crotonic acid.

Nevertheless despite this, one of the main disadvantages of memantine is its low solubility limiting its use in pharmaceutical formulations. Even though maybe partly overcome by use of the salts of memantine described above, the big majority of them is either not very useful or difficult to formulate, has physiological drawbacks or is only available in very specific formulations. In addition the acidic partners of the memantine in the salt are of no pharmaceutical value in themselves only adding - in some cases considerable - molecular weight to the active ingredient thus increasing the overall size of the pharmaceutical formulation without increasing the dosage. As in addition it is well-known that often there are a number of chemical difficulties to be overcome for obtaining salts of memantine, there still is a clear need for salts of memantine either
- being active in pain or even more active when compared to memantine base or hydrochloride salt; or
- being easily obtainable, or
- being easily cristalised, allowing more flexibility in formulating, or
- being highly soluble, especially if compared to memantine base, allowing better dissolution rates, especially if dissolving in an aqueous physiological surrounding, or
- having as acidic partner of the memantine a molecule having a beneficial pharmacological effect in itself, thus allowing for a highly efficient dose/weight relation of the active principle or
- having a synergistic effect in the combination of memantine and its acidic partner; or
- allowing the use of a lower therapeutic dose of either memantine and its acidic partner or of both.

Most desirably the salt should combine more than one, most preferably all of these advantages.

Besides memantine there are a considerable number of drugs known to be useful in the treatment or management of pain. Thus, for example opioids are frequently used as analgesics in pain, obtaining the analgesic effect through their action on morphinic receptors, preferably the µ-receptors. Besides these derivatives of morphine, there are a number of other well-known analgesics in the market.

One well-known group of analgesic compounds are the well established COX-INHIBITORs which include the NSAIDs (Non steroidal anti-inflammatory drugs) and have analgesic activity in a number of pain symptoms, with acetyl salicylic acid known under its trademark aspirin - despite being more than 100 years old - being an outstandingly used pharmaceutical. Besides Aspirin other COX-INHIBITORS whose use generally is also centered on anti-inflammatory action like ibuprofen, naproxen or diclofenac are among the worldwide most frequently applied pharmaceutical compounds. The basis of their activity is inhibition of cyclooxygenase (COX), one of the two activities of prostaglandine endoperoxide synthase (PGHS). It is a key enzyme in the prostaglandin pathway. For a number of COX-INHIBITORS the same problem as known for memantine base, a low solubility in water exists. As an example, this is especially true for the very popular and widely used and distributed members of the group of COX-INHIBITORS, naproxen, diclofenac and ibuprofen, whose poor solubility is a published fact, that has lead to considerably efforts for improvent by using solution enhancers etc. in their formulation. Accordingly the COX-INHIBITORS like naproxen, dixclofenac or ibuprofen hardly semed to be partners of choice for improving solubility of another also nearly insoluble compound.

Nevertheless, to its surprise the applicant has now found that memantine and COX-INHIBITORS having a carboxylic group can be combined to form a well-soluble mixed-salt.

Thus the object of the present invention is a salt of Memantine with a COX-INHIBITOR, wherein the COX-INHIBITOR has a carboxylic group.

These mixed salts are not only easily formed and crystallised they also considerable improve the solubility of memantine, but often also of its COX-INHIBITOR-partner. Also this association of the two active principles into the same salt exhibits several further advantages. Being linked as ion and counter-ion, they behave as a single chemical entity, thus facilitating the treatments, formulation, dosage etc. In addition to that, with both memantine and the COX-INHIBITOR being active analgesics these mixed salts are highly useful in the treatment of pain, especially also not losing any activity/weight by the addition of pharmacologically useless counterions. In addition the two active principles are complementing each other in the treatment especially of pain, but possibly also of various other diseases or symptoms. Thus, the mixed salts according to the invention do combine a high number of advantages over the state of the art.

The Applicant has further demonstrated the possibility to crystallise said salts. Even though also amorphous salts are also an aspect of the current invention, most preferred are crystalline salts. By that way the physico-chemical properties are improved. The formulation of the mixed salt is even easier with a solid to manipulate and an enhanced stability. The solubility, in particular the solubility of the memantine - but also in some cases like naproxen also of the COX-INHIBITOR salt - is also greatly augmented.

Another advantage is that the association of the two active principle into one unique species seems to allow for a better Pharmacokinetic/Pharmacodynamic (PKPD) including also a better penetration of the blood-brain barrier, which helps in the treatment of pain.

In general in most embodiments in which the salts of memantine are used (e.g. for the treatment of pain wtc.) these salts would be formulated into a convenient pharmaceutical formulation or a medicament. Accordingly a desirable advantage of a memantine salt, especially if crystallized, would show improved pharmaceutical properties and features, especially when compared to the free base or memantine hydrochloride. Thus, the memantine salt according to the invention, should desirably show at least one, preferably more, of the following features:
- to have a very small particle size, e.g. from 300 µm or lower; or
- to be and/or remain essentially free of agglomerates; or
- to be less or not very hygroscopic; or
- to allow by selection of the counter-ion of the memantine to help in formulating controlled release or immediate release formulations; or
- to have a high chemical stability; or
if given to a patient
- to decrease the inter- and intra-subject variability in blood levels; or
- to show a good absorption rate (e.g. increases in plasma levels or AUC); or
- to show a high maximum plasma concentration (e.g. Cₘₐₓ); or
- to show decreased time to peak drug concentrations in plasma (tₘₐₓ); or
- to show changes in half life of the compound (t_{1/2}), in whichever direction this change is preferably directed.

In one embodiment of the salt according to the invention the COX-INHIBITOR is selected from:
- Acetylsalicylic Acid;
- Triflusal;
- HTB (2-hydroxy-4-trifluoromethyl benzoic acid);
- Diflunisal;
- Meclofenamic acid;
- Mefenamic acid;
- Niflumic acid;
- Flufenamic acid.
- Diclofenac;
- Lonazolac;
- Acemetacin;
- Indomethacin;
- Tolmetin;
- Sulindac
- Etodolac;
- Keterolac
- Flurbiprofen;
- (RS)-Flurbiprofen;
- Esflurbiprofen;
- Ibuprofen;
- (RS)-Ibuprofen;
- S-(+)-Ibuprofen;
- Ketoprofen;
- (rac)-Ketoprofen
- R-(-)-Ketoprofen
- Bermoprofen;
- Pelubiprofen;
- Tenosal;
- Aceneuramic acid;
- Pirazolac;
- Xinoprofen;
- Flobufen;
- Anirolac;
- Zoliprofen;
- Bromfenac;
- Pemedolac;
- Dexpemedolac;
- Bindarit;
- Romazarit;
- Naproxen;
- (S)-Naproxen;
- Tiaprofenic acid;
- Ketorolac;
- Fenbufen;
- Fenoprofen;
- Flobufen; or
- Oxaprozin.

All of these COX-INHIBITORS are well-known and/or widely marketed drugs. In general all of these COX-Inhibitors which have at least one stereogenic center are to be understood as being included herein in their racemic form or as diastereoisomers or enantiomers or mixtures thereof.

In another embodiment of the salt according to the invention the COX-INHIBITOR is selected from:
- Salicylates,
- Anthranilates,
- Arylacetic Acids/ Arylalkanoic Acids,
- Arylpropionic Acids.

In another embodiment of the salt according to the invention the Salicylates are selected from:
- Acetylsalicylic Acid;
- Triflusal;
- HTB (2-hydroxy-4-trifluoromethyl benzoic acid); or
- Diflunisal;
preferably are
- Acetyl salicylic acid;
- HTB (2-hydroxy-4-trifluoromethyl benzoic acid); or
- Triflusal.

In another embodiment of the salt according to the invention the Anthranilates are selected from:
- Meclofenamic acid;
- Mefenamic acid;
- Niflumic acid; or
- Flufenamic acid.

In another embodiment of the salt according to the invention the Arylacetic Acids/Arylalkanoic Acids are selected from:
- Diclofenac;
- Lonazolac;
- Acemetacin;
- Indomethacin;
- Tolmetin; or
- Sulindac
- Etodolac;
- Keterolac
, preferably from
- Diclofenac;
- Lonazolac;
- Acemetacin;
- Indomethacin;
- Tolmetin; or
- Sulindac
, most preferably is
- Diclofenac.

In another embodiment of the salt according to the invention the Arylpropionic Acids are selected from:
- Flurbiprofen;
- (RS)-Flurbiprofen;
- Esflurbiprofen;
- Ibuprofen;
- (RS)-Ibuprofen;
- S-(+)-Ibuprofen;
- Ketoprofen;
- (rac)-Ketoprofen;
- R-(-)-Ketoprofen;
- Naproxen;
- (S)-Naproxen;
- Tiaprofenic acid;
- Ketorolac;
- Fenbufen;
- Fenoprofen;
- Flobufen;
- Oxaprozin;
- Tolmetin;
- Xinoprofen;
- Flobufen;
- Zoliprofen;
- Bermoprofen; or
- Pelubiprofen;
, preferably from
- Flurbiprofen;
- (RS)-Flurbiprofen;
- Esflurbiprofen;
- Ibuprofen;
- (RS)-Ibuprofen;
- S-(+)-Ibuprofen;
- Ketoprofen;
- (rac)-Ketoprofen;
- R-(-)-Ketoprofen;
- Naproxen;
- (S)-Naproxen;
- Tiaprofenic acid; or
- Ketorolac;
preferably is
- (RS)-Flurbiprofen;
- Esflurbiprofen;
- (RS)-Ibuprofen;
- S-(+)-Ibuprofen;
- (rac)-Ketoprofen;
- R-(-)-Ketoprofen; or
- (S)-Naproxen.

Another embodiment is a salt of Memantine with an COX-INHIBITOR according to the invention selected from Memantine-Ibuprofen salt, Memantine-Flurbiprofen salt, Memantine-Diclofenac salt, Memantine-Acetylsalicylic Acid salt, Memantine-(S)-Naproxen salt, Memantine/Triflusal salt, or Memantine/2-hydroxy-4-trifluoromethyl benzoic acid (HTB) salt.

Another embodiment of the invention is a memantine-Ibuprofen salt.

Another embodiment of the invention is a memantine-Diclofenac salt.

Another embodiment of the invention is a memantine-Acetylsalicylic Acid salt.

Another embodiment of the invention is a memantine-(S)-Naproxen salt.

Another embodiment of the invention is a memantine-Flurbiprofen salt.

Another embodiment of the invention is a memantine-Triflusal salt.

Another embodiment of the invention is a memantine-HTB salt.

As the applicant has shown the possibility to crystallise said salts according to the invention a crystalline form of a salt according to the invention it is a separate, highly intersting aspect of the current invention.

Another embodiment the present invention relates to a process for the production of a salt according to the invention as described above comprising the steps of:
dissolving a COX-INHIBITOR with a carboxylic group either as a free acid or as a salt together with, or after, or before, memantine either as a free base or as a salt in an organic solvent,
stirring the mixture obtained at a temperature between 0° C and 80°C, evaporating the solvent and/or evaporating the solvent, and drying of the resulting product.

Preferably in the process above
- the organic solvent is selected from acetone, acetonitrile, isobutyl acetate, hepatane, methanol, tetrahydrofuran, isopropanol, ethanol or cyclohexane; and/or
- the solvent is evaporated under high vacuum; and/or
- the ratio of memantine to COX-INHIBITOR is 1:1 to 1:2, preferably is 1:1; and/or
- the memantine dissolved is a free base.

Both parts of the salt are well-known drugs sometimes used for a long time worldwide. Due to the therapeutic interest in memantine in the treatment of pain symptoms like diabetic neuropathy and the well-known properties of COX-INHIBITORS in this field of medical indication, a further object of the present invention is a medicament containing a memantine-COX-INHIBITOR salt, or its crystalline form according to the invention.

Thus the invention also concerns a medicament comprising at least one salt according to the invention as described above (or in preferred aspects as will be described below) and optionally one or more pharmaceutically acceptable excipients.

A further object of the invention is a pharmaceutical composition **characterized in that** it comprises an efficient amount of at least one salt according to the invention as described above (or in preferred aspects as will be described below) or its crystalline form, in a physiologically acceptable medium.

The medicament according to the present invention may be in any form suitable for the application to humans and/or animals, preferably humans including infants, children and adults and can be produced by standard procedures known to those skilled in the art. The medicament of the present invention may for example be administered parentally, including intramuscular, intraperitoneal, or intravenous injection; or orally, including administration as tablets, pellets, granules, capsules, lozenges, aqueous or oily solutions, suspensions, emulsions, sprays or as reconstituted dry powdered form with a liquid medium.

Typically, the medicaments according to the present invention may contain 1-60 % by weight of one or more of the salts or their crystalline form as defined herein and 40-99 % by weight of one or more auxiliary substances (additives/excipients).

The compositions of the present invention may also be administered topically or via a suppository.

The daily dosage for humans and animals may vary depending on factors that have their basis in the respective species or other factors, such as age, sex, weight or degree of illness and so forth. The daily dosage for humans preferably is in the range of 10 to 2000 milligrams of active substance to be administered during one or several intakes per day.

A further aspect of the invention relates to the use of a salt according to the invention as described above (or in preferred aspects as will be described below) for the treatment of pain, preferably acute pain, chronic pain, neuropathic pain, hyperalgesia, allodynia or cancer pain, including diabetic neuropathy or osteoarthritis. Preferably this use is provided for in form of a medicament or a pharmaceutical composition according to the invention as described above.

Another object of the current invention is a method of treatment of pain, preferably acute pain, chronic pain, neuropathic pain, hyperalgesia, allodynia or cancer pain, including diabetic neuropathy or osteoarthritis, by providing to a patient in need thereof a sufficient amount of a salt according to the invention as described above (or in preferred aspects as will be described below). Preferably a salt according to the invention or its crystalline form according to the invention is provided in physiologically suitable form like e.g. in form of a medicament or a pharmaceutical composition according to the invention as described above.

An interesting COX-INHIBITOR to be combined with memantine is the marketed drug naproxen, whose chemical name is 2(S)-(6-methoxy-2-naphtyl)propionic acid, and which is also described as a physiologically acceptable salt. It has an empirical formula of C₁₄H₁₄O₃, an Mp of 153°C and a pKa of 4.2.

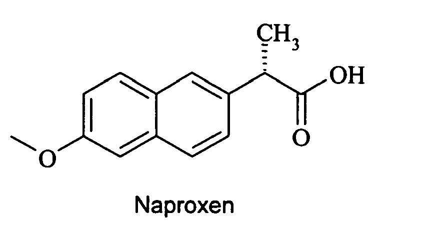

Thus, another very preferred aspect of the invention relates to a Memantine-(S)-Naproxen salt.

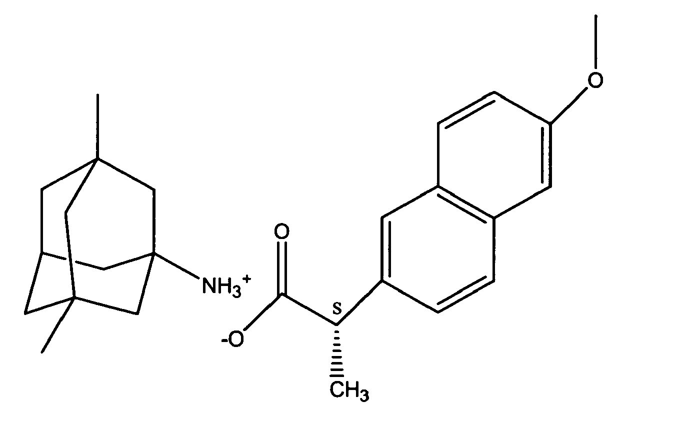

A second object of this preferred aspect of the invention is a crystalline form of a Memantine-(S)-Naproxen salt.

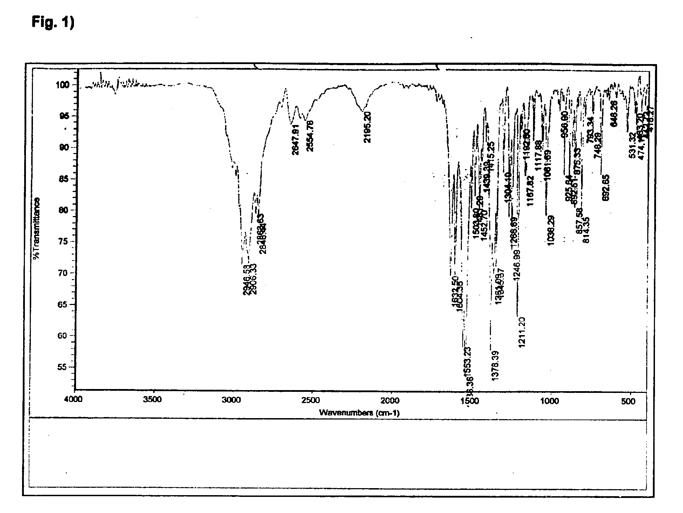

More particularly, the invention concerns a memantine-(S)-naproxene salt or a crystalline form of memantine-(S)-naproxene salt, **characterized in that** it shows a Fourier Transform Infra Red pattern with absorption bands at 2947, 2906, 2864, 2848, 2648, 2555, 2195, 1633, 1604, 1553, 1536, 1378, 1361, 1346, 1247, 1211, 1036, 858, 814, and 693 cm⁻¹ (see figure 1).

The invention also encompasses a memantine-(S)-naproxene salt or a crystalline form of memantine-(S)-naproxen salt with a ¹H NMR spectrum of figure 2, in D4-methanol at 400 MHz.

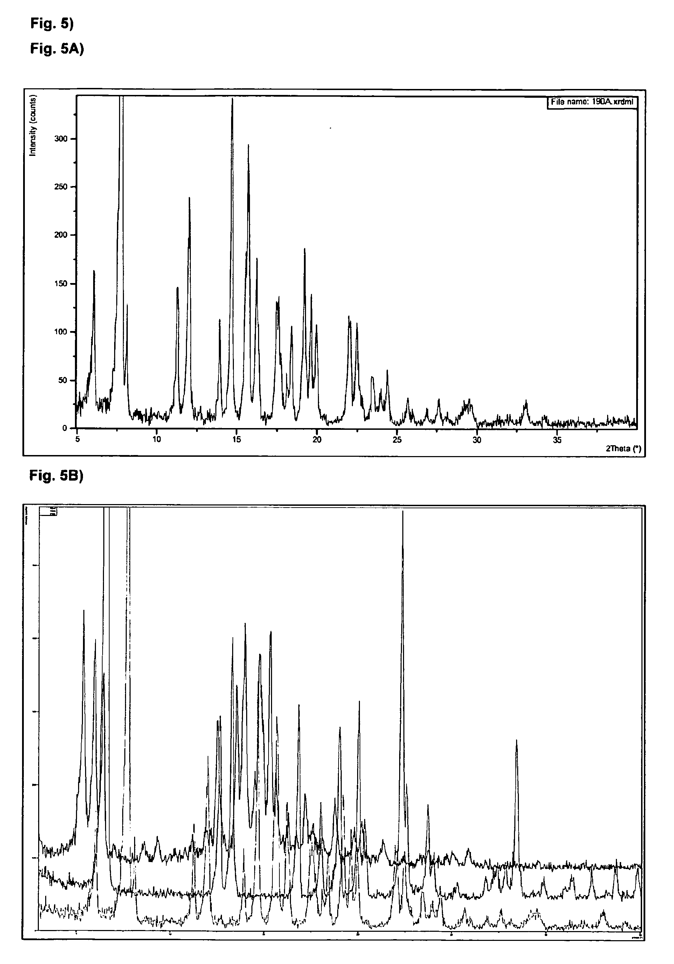

In another embodiment, the invention concerns a crystalline form of memantine-(S)-naproxen salt, **characterized in that** it shows a X-Ray powder diffraction pattern as disclosed in figure 5. In another embodiment, the present invention concerns a crystalline form of memantine-(S)-naproxen salt, **characterized in that** it crystallizes as a monoclinic unit cell with the following dimensions:
- a = 24.34 Å
- b = 6.65 Å
- c = 15.63 Å
- β angle of 109.18°

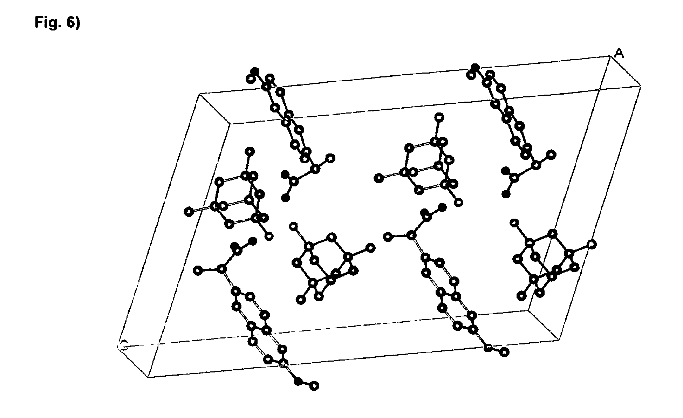

The single crystals are shown in Fig. 6).

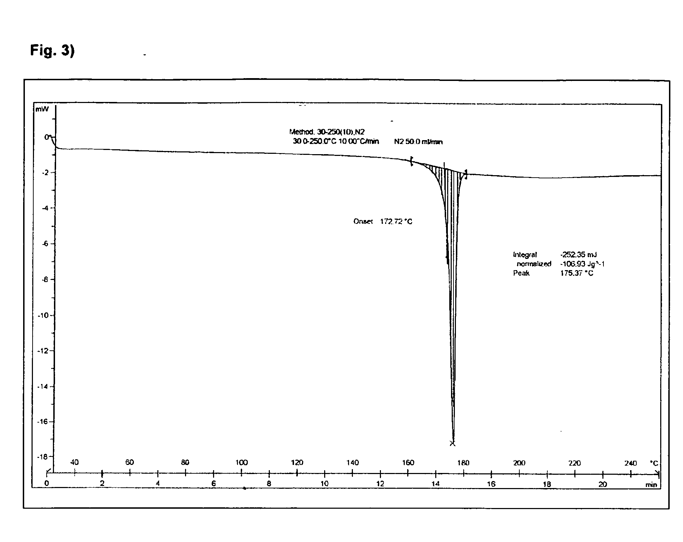

The crystalline form of memantine-(S)-naproxen salt according to the present invention is **characterized in that** the endothermic sharp peak corresponding to the melting point has an onset at 173 °C, measured by DSC analysis (10°C/min), see figure 3.

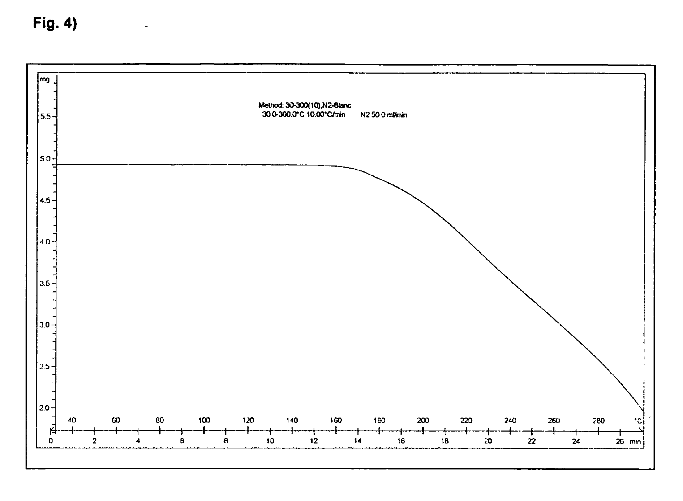

The TG analysis of the crystalline form according to the invention shows no weight loss at temperatures lower than the melting point (see figure 4).

Another interesting COX-INHIBITOR to be combined with memantine for the use according to the invention is the marketed drug Triflusal (2-Acetoxy-4-trifluoromethyl-benzoic acid). Triflusal - having an empirical formula of C₁₀H₇F₃O₄ has a Mp of 116°C and a pKa of 3.34. It has the following formula:

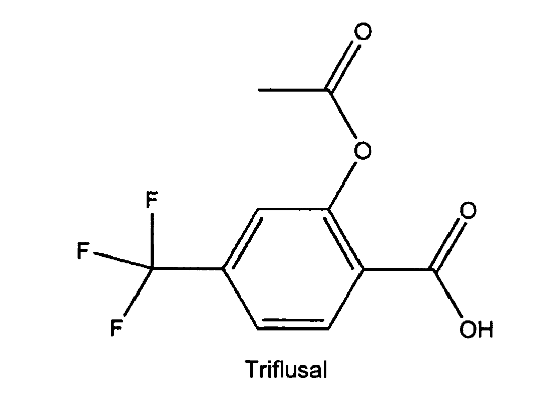

Thus, a very preferred aspect of the invention relates to a Memantine-Triflusal salt or a salt of Memantine with HTB (2-hydroxy-4-trifluoromethyl benzoic acid), a metabolite of Triflusal.

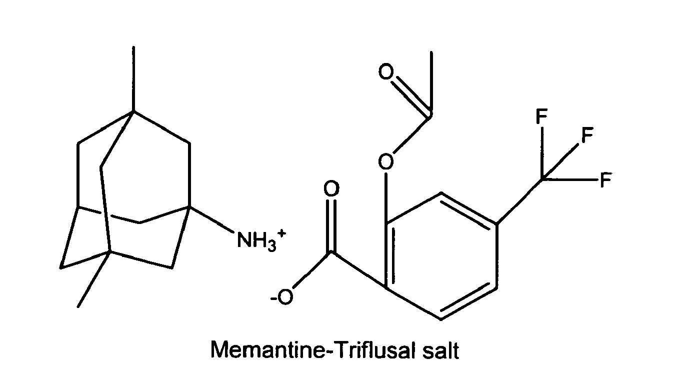

The Applicant has further demonstrated the possibility to crystallise said salts. By that way the physico-chemical properties are improved. The formulation of the mixed salt is even easier with a solid to manipulate and an enhanced stability. The solubility, in particular the solubility of the memantine is also greatly augmented.

A further object of this invention is a crystalline form of a Memantine- Triflusal salt.

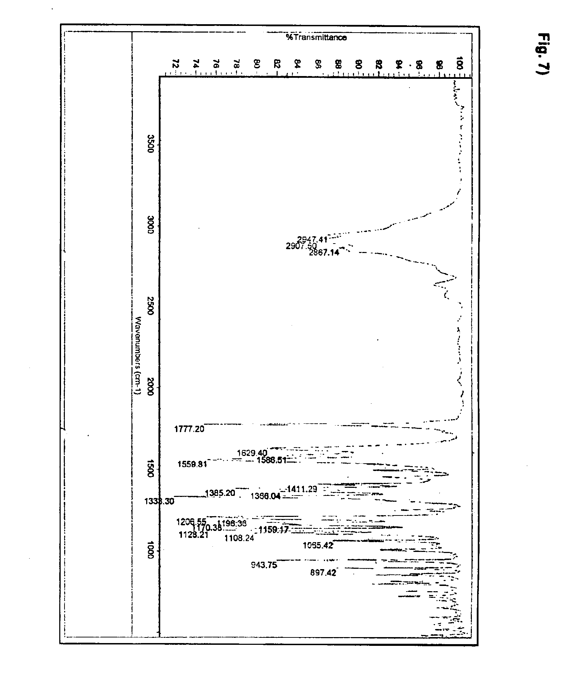

More particularly, the invention concerns a memantine-Triflusal salt or a crystalline form of memantine-triflusal salt, **characterized in that** it shows a Fourier Transform Infra Red pattern with absorption bands at 2947, 2908, 2867, 1777, 1629, 1587, 1560, 1411, 1385, 1366, 1333, 1207, 1196, 1170, 1159, 1128, 1108, 1065, 944, and 897 cm⁻¹ (see figure 7).

The invention also encompasses a memantine-Triflusal salt or a crystalline form of memantine- Triflusal salt with a ¹H NMR spectrum of figure 8, in D4-chloroform at 400 MHz.

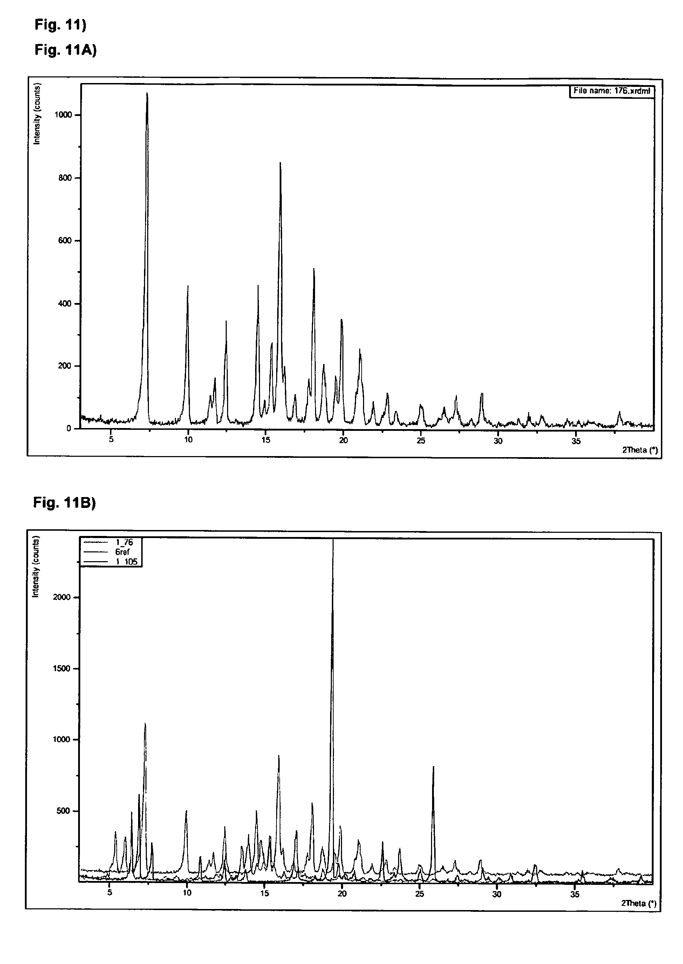

In another embodiment, the invention concerns a crystalline form of memantine-Triflusal salt, **characterized in that** it shows a X-Ray powder diffraction pattern as disclosed in figure 11.

In another embodiment, the present invention concerns a crystalline form of memantine-Triflusal salt, **characterized in that** it crystallizes as a monoclinic unit cell with the following dimensions:
- a = 30.96 Å
- b = 13.62 Å
- c = 11.94 Å
- β angle of 112.34°

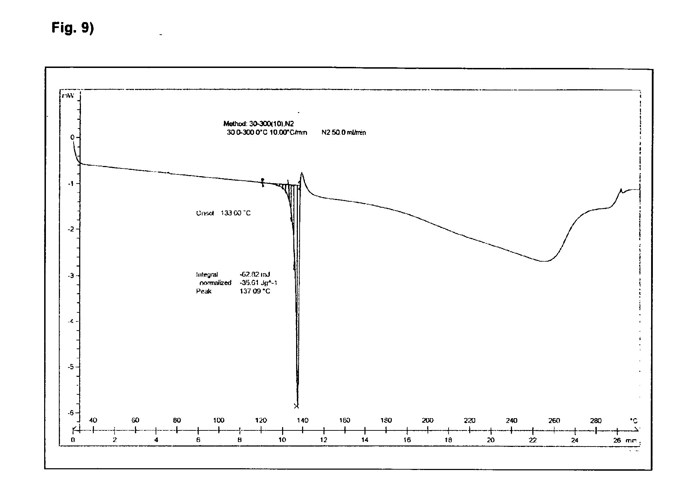

The crystalline form of memantine-Triflusal salt according to the present invention is **characterized in that** the endothermic sharp peak corresponding to the melting point has an onset at 133 °C, measured by DSC analysis (10°C/min), see figure 9.

Another further aspect of the invention is a crystalline form of a Memantine/HTB salt.

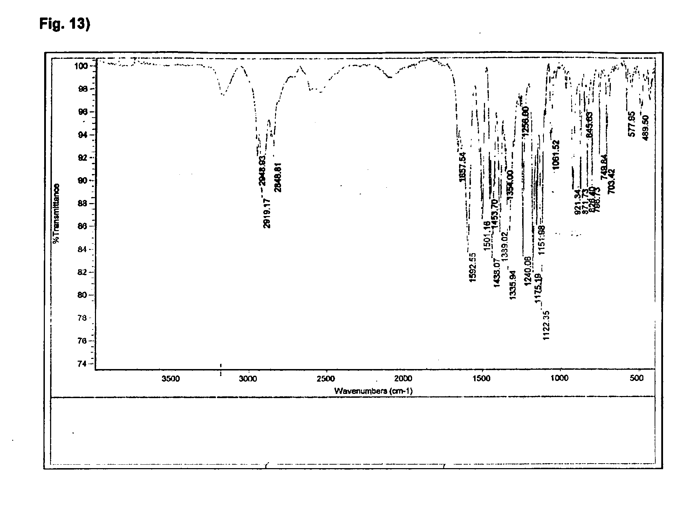

More particularly, the invention concerns a memantine/HTB salt or a crystalline form of memantine/HTB salt, **characterized in that** it shows a Fourier Transform Infra Red pattern with absorption bands at 2949, 2919, 2849, 1668, 1593, 1501, 1454, 1438, 1389, 1354, 1336, 1256, 1240, 1175, 1152, 1122, 1062, 921, 972, 846, 828, 797, 750, 703, 578 and 490 cm⁻¹ (see figure 13).

The invention also encompasses a memantine/HTB salt or a crystalline form of memantine/HTB salt with a ¹H NMR spectrum of figure 14, in D4-chloroform at 400 MHz.

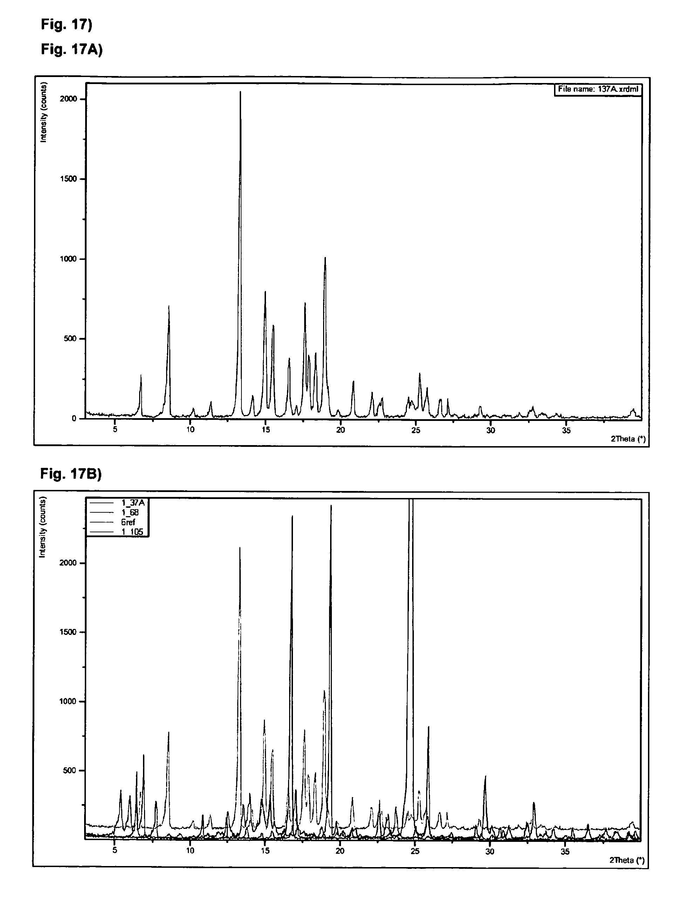

In another embodiment, the invention concerns a crystalline form of memantine/HTB salt, **characterized in that** it shows a X-Ray powder diffraction pattern as disclosed in figure 17.

In another embodiment, the present invention concerns a crystalline form of memantine/HTB salt, **characterized in that** it crystallizes as a triclinic unit cell with the following dimensions:
- a=7.13 Å
- b = 11.09 Å
- c = 13.55 Å
- α angle of 94.45°
- β angle of 94.77°
- γ angle of 108.37°.

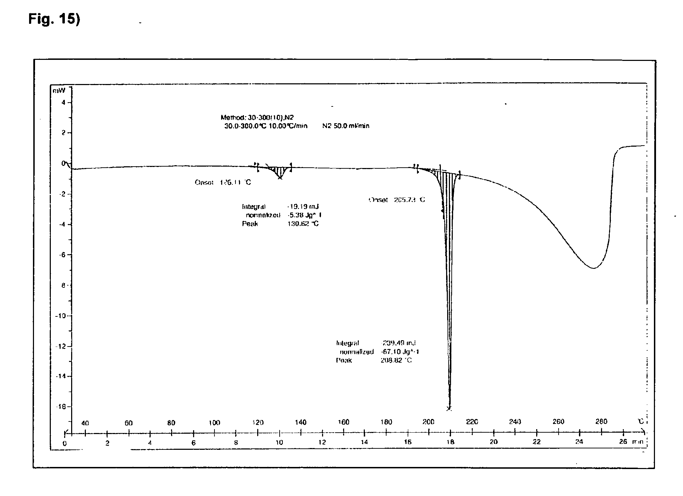

The crystalline form of memantine/HTB salt according to the present invention is **characterized in that** the endothermic sharp peak corresponding to the melting point has an onset at 206 °C, measured by DSC analysis (10°C/min), see figure 15.

The present invention is illustrated below with the help of the following figures and examples. These illustrations are given solely by way of example and do not limit the invention.

### Brief description of the figures :

**Figure 1** **: FT-IR spectrum of crystalline form of Memantine-(S)-Naproxen salt.**
   The FTIR spectrum of the crystalline form of Memantine-(S)-Naproxen salt is shown measured as described in Example 1.
**Figure 2** **:** **¹*****H-NMR* spectrum of crystalline form of Memantine-(S)-Naproxen salt.**
   The ¹H-NMR spectrum of the crystalline form of Memantine-(S)-Naproxen salt is shown measured as described in Example 1.
**Figure 3****: DSC analysis of crystalline form of Memantine-(S)-Naproxen salt.**
   The DSC analysis of the crystalline form of Memantine-(S)-Naproxen salt is shown measured as described in Example 1.
**Figure 4****: TG analysis of crystalline form of Memantine-(S)-Naproxen salt.**
   The TG analysis of the crystalline form of Memantine-(S)-Naproxen salt is shown measured as described in Example 1.
**Figure 5****: Powder X-Ray diffraction pattern of crystalline form of Memantine-(S)-Naproxen salt (XRPD) (****Fig. 5A****). Comparison to XRPD of the starting products memantine base and triflusal acid (****Fig. 5B****) and (S)-Naproxen (****Fig. 5B****)**
   The Powder X-Ray diffraction pattern of the crystalline form of Memantine-(S)-Naproxen salt is shown measured as described in Example 1. Also the comparison between this XRPD and the XRPD of the starting products memantine base and (S)-Naproxen is shown.
**Figure 6****: Crystal structure of Memantine-(S)-Naproxen salt**
   The crystal structure as calcultated from the single crystal X-ray diffraction is given.
**Figure 7****: FT-IR spectrum of crystalline form of Memantine-Triflusal salt**
   The FTIR spectra of the crystalline form of Memantine-Triflusal salt are shown measured as described in Example 3.
**Figure 8****:** **¹****H-NMR spectrum of crystalline form of Memantine-Triflusal salt**
   The ¹H-NMR spectrum of the crystalline form of Memantine-Triflusal salt are shown measured as described in Example 3.
**Figure 9****: DSC analysis of crystalline form of Memantine-Triflusal salt**
   The DSC analysis of the crystalline form of the Memantine-Triflusal salt are shown measured as described in Example 3.
**Figure 10****: TG analysis of crystalline form of Memantine-Triflusal salt**
   The TG analysis of the crystalline form of the Memantine-Triflusal salt are shown measured as described in Example 3.
**Figure 11****: Powder X-Ray diffraction pattern of crystalline form of Memantine-Triflusal salt (XRPD) (Fig. 10A). Comparison to XRPD of the starting products memantine base and triflusal acid (Fig. 10B)**
   The Powder X-Ray diffraction pattern of the crystalline form of the Memantine-Triflusal salt is shown measured as described in Example 1. Also the comparison between this XRPD and the XRPD of the starting products memantine base and triflusal acid is shown.
**Figure 12****: Crystal structure of Memantine-Triflusal salt**
   The crystal structure as calcultated from the single crystal X-ray diffraction is given.
**Figure 13****: FT-IR spectrum of crystalline form of Memantine-HTB salt**
   The FTIR spectra of the crystalline form of Memantine-HTB salt are shown measure as described in Example 5.
**Figure 14****:** **¹****H-NMR spectrum of crystalline form of Memantine-HTB salt**
   The ¹H-NMR spectrum of the crystalline form of Memantine-HTB salt are shown measure as described in Example 5.
**Figure 15****: DSC analysis of crystalline form of Memantine-HTB salt**
   The DSC analysis of the crystalline form of Memantine-HTB salt are shown measure as described in Example 5.
**Figure 16****: TG analysis of crystalline form of Memantine-HTB salt**
   The TG analysis of the crystalline form of Memantine-HTB salt are shown measure as described in Example 5.
**Figure 17****: Powder X-Ray diffraction pattern of crystalline form of Memantine-HTB salt (XRPD) (****Fig. 17A****). Comparison to XRPD of the starting products memantine base and HTB (****Fig. 17B****)**
   The Powder X-Ray diffraction pattern of the crystalline form of Memantine-HTB salt is shown measure as described in Example 5. Also the comparison between this XRPD and the XRPD of the starting products memantine base and HTB is shown.
**Figure 18****: Single crystals of the crystalline form of Memantine-HTB salt**
   Under microscope single crystals are shown obtained in good quality from a process according to example 5.
**Figure 19****: Crystal structure of the Memantine-HTB salt**
   The crystal structure as calcultated from the single crystal X-ray diffraction is given.

### EXAMPLE

### Example 1: Preparation of Memantine-(S)-naproxen salt.

To an assay tube containing Naproxen (100 mg, 0.43 mmol) dissolved in methanol (1.4 mL), was added at room temperature 1,3-dimethyl-5-aminoadamantane (78 mg, 0.43 mmol, 1 eq.) diluted with methanol (1 mL). A complete dissolution was obtained in an exothermic reaction. The solvent was evaporated without stirring at room temperature under atmospheric pressure. After complete evaporation, the salt of Memantine - Naproxen 1:1 was obtained as white crystals (178 mg, quantitative yield).

Good quality single crystals were obtained.

This product was fully characterized by ¹HNMR, FTIR, X-Ray diffraction, and melting point (see figures 1 to 5).

### FT-IR spectrum (see Fig. 1)

The FTIR spectra were recorded using a Thermo Nicolet Nexus 870 FT-IR, equipped with a beamsplitter KBr system, a 35 mW He-Ne laser as the excitation source and a DTGS KBr detector. The spectra were acquired in 32 scans at a resolution of 4 cm⁻¹.

The sample (KBr pellets) shows a Fourier Transform Infra Red spectrum with absorption bands at 2946.6 (m), 2906.3 (m), 2863.6 (m), 2848.4 (m), 1632.5 (m), 1604.4 (m), 1553.2 (s), 1536.4 (s), 1378.4 (s), 1211.2 (s), 1036.3 (w), 857.6 (w), 814.35 (w), (see figure 1).

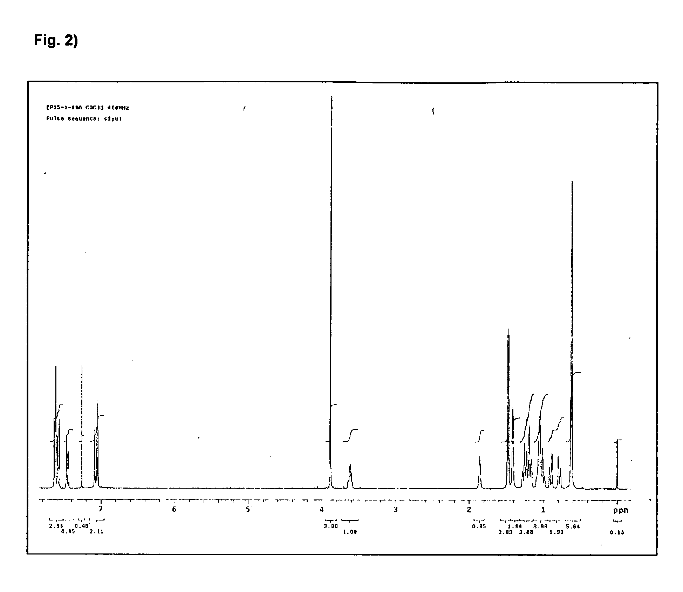

### ¹H-NMR spectrum (see Fig. 2)

Proton nuclear magnetic resonance analyses were recorded in deuterated chloroform (CDCl₃) in a Varian Mercury 400 spectrometer, equipped with a broadband probe ATB 1H/19F/X of 5 mm. Spectra were acquired dissolving 5-10 mg of sample in 0.6 mL of deuterated solvent.

¹H NMR spectrum (see figure 2), in D-chloroform at 400 MHz shows peaks at 7.63 (d, *J* = 8.6 Hz, 1 H) ; 7.62 (s, 1 H) ; 7.58 (d, *J* = 8.6 Hz, 1 H) ; 7.46 (d, *J* = 8.6 Hz, 1 H) ; 7.10-7.02 (m, 2H) ; 3.88 (s, 3H) ; 3.60 (q, *J* = 7.2 Hz, 1 H) ; 1.90-1.83 (m, 1 H) ; 1.47 (d, *J* = 7.2 Hz, 3H) ; 1.41 (s, 2H); 1.32-1.13 (m, 4H); 1.12-0.96 (m, 4H) ; 0.91 (d, *J* = 12.4 Hz, 1 H) ; 0.79 (d, *J* = 12.4 Hz, 1H); 0.63 (s, 6H)

### DSC analysis (see Fig. 3)

DSC analyses were recorded with a Mettler DSC822^{e}. A sample of 2.3600 mg was weighed into a 40 µL aluminium crucible with a pinhole lid and was heated, under nitrogen (50 mL/min), at 10 °C/min from 30 to 250 °C.

The novel type of crystal of the present invention (Form III) is **characterized in that** the endothermic sharp peak corresponding to the melting point has an onset at 172.72 °C (fusion enthalpy -106.93 J/g), measured by DSC analysis (10 °C/min), see figure 3.

### TG analysis (see Fig. 4)

Thermogravimetric analyses were recorded with a thermogravimetric analyzer Mettler TGA/SDTA851^{e}. A sample of 4.9178 mg was weighed into a 70 µL alumina crucible with a pinhole lid, and was heated at 10 °C/min from 30 to 300 °C, under nitrogen (50 mL/min).

The TG analysis of crystalline Form III according to the invention shows no weight loss at temperatures lower than the melting point (see figure 4).

### Powder X-Ray diffraction pattern (XRPD) (Fig. 5A)

XRPD analysis was performed using a Philips X'Pert diffractometer with Cu Kα radiation in Bragg-Brentano geometry. The system is equipped with a proportional detector. The measurement parameters were as follows: the range of 2θ was 3° to 40° at a scan rate of 1,8° per minute (see figure 5A).

List of selected peaks:

| **2θ (°)** | **d (Å)** | **I (%)** |
|---|---|---|
| 6.06 | 14.58 | 15 |
| 7.77 | 11.38 | 100 |
| 8.13 | 10.88 | 9 |
| 11.29 | 7.84 | 8 |
| 12.03 | 7.36 | 12 |
| 13.96 | 6.34 | 4 |
| 14.69 | 6.03 | 14 |
| 15.73 | 5.64 | 11 |
| 16.22 | 5.47 | 6 |
| 17.48 | 5.08 | 5 |
| 18.13 | 4.89 | 1 |
| 18.43 | 4.82 | 3 |
| 19.24 | 4.61 | 6 |
| 19.63 | 4.52 | 4 |
| 19.96 | 4.45 | 3 |
| 22.05 | 4.03 | 3 |
| 22.46 | 3.96 | 2 |
| 23.45 | 3.79 | 1 |
| 24.42 | 3.65 | 1 |

In addition the Powder X-Ray diffraction pattern of the starting products memantine base and (S)-naproxen were compared to the XRPD above (Fig. 5B), proving formation of the salt.

### Example 2: Single Crystal XRD analysis of a single crystal derived from example 1

The crystal structure was determined from single crystal X-ray diffraction data. The colourless prism (0.45 x 0.20 x 0.08 mm) used was obtained from the preparation according to example 1. Analysis was performed using a Bruker Smart Apex diffractometer with graphic monochromated Mo_{Kα} radiation equipped with a CCD detector. No significant decay of standard intensities were observed. Data reduction (Lorentz and polarization corrections) and absorption correction were applied (program used: SAINT 5.0). The structure was solved with direct methods and least-squares refinement of Fₒ² against all measured intensities was carried out (program used: SHELXTL -NT 6.1). All non-hydrogen atoms were refined with anisotropic displacement parameters.

Relevant structural data:

| Crystal system | Monoclinic |
|---|---|
| Space group: | *C*2 |
| a (Å) | 24.341 (3) |
| b (Å) | 6.6538 (7) |
| c (Å) | 15.6312 (15) |
| β (°) | 109.176 (2) |
| Z | 4 |
| D calc. (Mg/m³) | 1.138 |
| N. of refl. | 5080 |
| Refl. with I>2σ(I) | 3404 |
| R (I> 2σ(I)) | 0.0701 |

Also from these single crystals powder diffraction patterns were acquired as described for example 1. The positions of the peaks coincided with those in Figure 5 indicating that the product obtained according to example 1 only gives a crystalline phase.

### Example 3: Preparation of Memantine-triflusal salt.

To an assay tube with magnetic stirring containing Triflusal (153 mg, 0.616 mmol, 1.1 eq.) in isobutyl acetate (1 mL), was added dropwise over 5 min at room temperature 1,3-dimethyl-5-aminoadamantane (100 mg, 0.56 mmol) diluted with isobutyl acetate (1 mL)., On the onset of precipitation, the mixture was stirred for 30 min at room temperature. The white solid was filtered with a sintered funnel (porosity 3) and washed with isobutyl acetate (0.8 mL). After drying at room temperature under vacuum, the salt Memantine - Triflusal 1:1 was obtained as a white solid (132 mg, 52 % yield).

The product has been fully characterized by ¹HNMR, FTIR, X-Ray diffraction, and melting point (see figures 7 to 11).

### FT-IR spectrum (see Fig. 7).

The FTIR spectra were recorded using a Thermo Nicolet Nexus 870 FT-IR, equipped with a beamsplitter KBr system, a 35 mW He-Ne laser as the excitation source and a DTGS KBr detector. The spectra were acquired in 32 scans at a resolution of 4 cm⁻¹.

The sample (KBr pellets) shows a Fourier Transform Infra Red spectrum with absorption bands at 2947.4 (m), 2907.5 (m), 2867.1 (m), 1777.2 (s), 1629.4 (m), 1586.5 (m), 1559.8 (s), 1385.2 (s), 1333.3 (s), 1206.5 (s), 1128.2 (s), 1108.2 (s), 943.7 (m) (see figure 7).

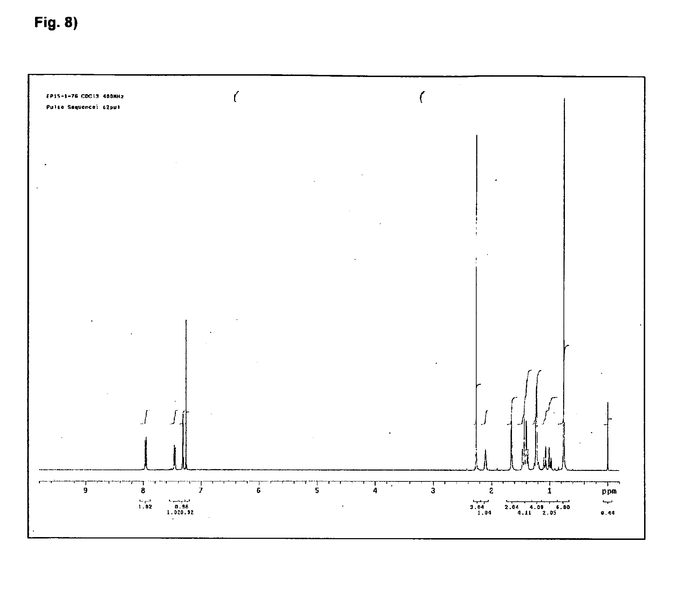

### ¹H-NMR spectrum (see Fig. 8).

Proton nuclear magnetic resonance analyses were recorded in deuterated chloroform (CDCl₃) in a Varian Mercury 400 NMR spectrometer, equipped with a broadband probe ATB 1H/19F/X of 5mm. Spectra were acquired solving 5 - 10 mg of sample in 0.6 mL of deuterated solvent.

¹H NMR spectrum (see figure 8), in D-chloroform at 400 MHz shows peaks at 7.96 (d, *J* = 8.1Hz, 1 H) ; 7.46 (dd, *J* = 1.0 Hz, *J* = 8.1 Hz, 1 H) ; 7.32 (d, *J* = 1.0 Hz, 1 H) ; 2.27 (s, 3H) ; 2.14-2.06 (m, 1 H) ; 1.66 (s, 2H) ; 1.45 (d, *J* = 11.5 Hz, 2H ) ; 1.39 (d, *J* = 11.5 Hz, 2H ) ; 1.29-1.17 (m, 4H) ; 1.09 (d, *J* = 12.5 Hz, 1H) ; 0.99 (d, *J* = 12.5 Hz, 1H) ; 0.76 (s, 6H).

### DSC analysis (see Fig. 9)

DSC analyses were recorded in a Mettler Toledo DSC822e. Samples of 1-2 mg were weighted into 40 µL aluminium crucibles with a pinhole lid, and were heated under nitrogen (50 mUmin) at 10°C/min from 30 to 300°C.

The crystal shows an endothermic sharp peak corresponding to the melting point has an onset at 133.00 °C (fusion enthalpy -35.61 J/g), measured by DSC analysis (10 °C/min), see figure 9.

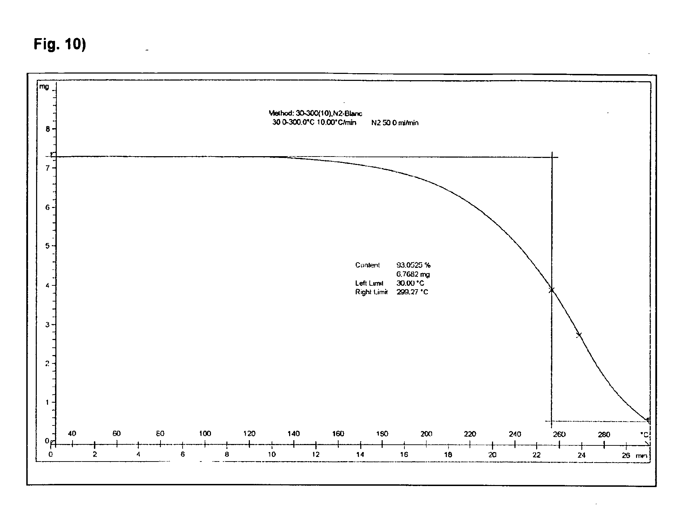

### TG analysis (see Fig. 10).

Thermogravimetric analyses were recorded in a thermogravimetric analyzer Mettler TGA/SDTA851e. Samples of 7 - 8 mg were weighted into 70 µL aluminium crucibles with a pinhole lid, and heated at 10°C/min from 30 to 300°C, under nitrogen (50 mL/min).

The TG analysis of the crystalline form according to the invention shows no weight loss at temperatures lower than the melting point (see figure 10).

### Powder X-Ray diffraction pattern (XRPD) (see Fig. 11A)

XRPD analysis was performed using a Philips X'Pert diffractometer with Cu K_{α} radiation in Bragg-Brentano geometry. The system is equipped with a proportional detector. The measurement parameters were as follows: the range of 2θ was 3° to 40° at a scan rate of 1.8° per minute (see figure 11A).

List of selected peaks:

| **2θ (°)** | **d (Å)** | **I (%)** |
|---|---|---|
| 7.30 | 12.11 | 100 |
| 9.97 | 8.88 | 29 |
| 11.41 | 7.76 | 5 |
| 11.73 | 7.55 | 8 |
| 12.48 | 7.10 | 18 |
| 14.52 | 6.10 | 21 |
| 14.95 | 5.93 | 4 |
| 15.38 | 5.76 | 12 |
| 15.94 | 5.56 | 36 |
| 16.22 | 5.46 | 8 |
| 16.90 | 5.25 | 4 |
| 17.75 | 5.00 | 5 |
| 18.09 | 4.90 | 19 |
| 18.73 | 4.74 | 7 |
| 19.52 | 4.55 | 5 |
| 19.88 | 4.47 | 12 |
| 20.81 | 4.27 | 4 |
| 21.08 | 4.22 | 8 |
| 21.97 | 4.05 | 2 |
| 22.87 | 3.89 | 3 |
| 23.41 | 3.80 | 1 |
| 25.05 | 3.55 | 2 |
| 26.48 | 3.37 | 2 |
| 27.28 | 3.27 | 3 |
| 28.91 | 3.09 | 3 |

In addition the Powder X-Ray diffraction pattern of the starting products memantine base and triflusal acid were compared to the XRPD above (Fig. 11 B), proving the formation of the salt.

### Example 4: Single Crystal XRD analysis of a single crystal of memantine-triflusal salt.

Crystal structure of memantine-triflusal salt (1:1) has been determined from single crystal X-ray diffraction data. The colourless prism (0.34 × 0.10 × 0.04 mm) used were obtained from a liquid-liquid diffusion crystallisation (chloroform-diethyl ether) with equimolar amounts of Memantine and Triflusal.

Analysis was performed at room temperature using an Oxford Diffraction Xcalibur diffractometer with graphite monochromated Cu K_{α} radiation equipped with a CCD detector. Data were collected using phi and omega scans (program used: CrysAlis CCD 1.171.32.5). No significant decay of standard intensities was observed. Data reduction (Lorentz and polarization corrections) and absorption correction were applied (program used: CrysAlis CCD 1.171.32.5). The structure was solved with direct methods and least-squares refinement of Fₒ² against all measured intensities was carried out (programs used: SIR2006 and SHELXL97). All non-hydrogen atoms were refined with anisotropic displacement parameters.

Relevant structural data:

| Crystal system | Monoclínic |
|---|---|
| Space group | *C*2/c |
| a (Å) | 30.955(4) |
| b (Å) | 13.6184(18) |
| c (Å) | 11.9378(9) |
| β (°) | 112.344(7) |
| Volume (Å³) | 4654.6(9) |
| Z | 8 |
| D calc. (Mg/m³) | 1.220 |
| N. of refl. | 3510 |
| Refl. with I > 2σ(I) | 968 |
| R (I > 2σ(I)) | 0.0613 |

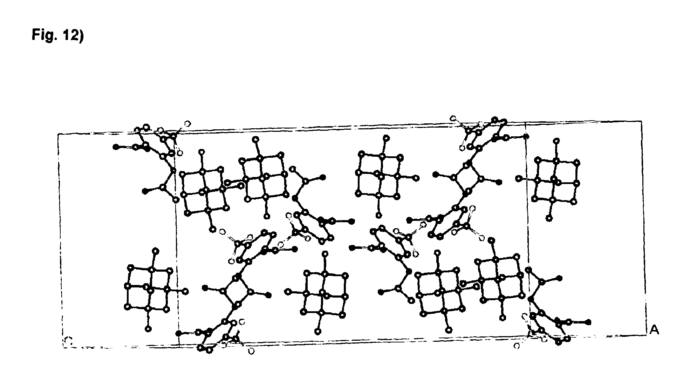

The crystal structure is depicted in figure 12.

Simulation of XRPD diffractogram from single crystal data gives an almost identical diagram to the experimental one presented above (Figure 11), indicating that the product obtained according to example 3 only gives one crystalline phase.

### Example 5: Preparation of Memantine-HTB salt.

To an assay tube containing 1,3-dimethyl-5-aminoadamantane (100 mg, 0.56 mmol) diluted with methanol (0.4 mL) was added at room temperature Triflusal (138 mg, 0.56 mmol, 1 eq.) resulting in complete dissolution (exothermic reaction). The solvent was evaporated slowly without stirring at room temperature or at 0 °C under atmospheric pressure. After complete evaporation, the salt Memantine - HTB 1:1 was obtained as colorless needles (238 mg, quantitative yield).

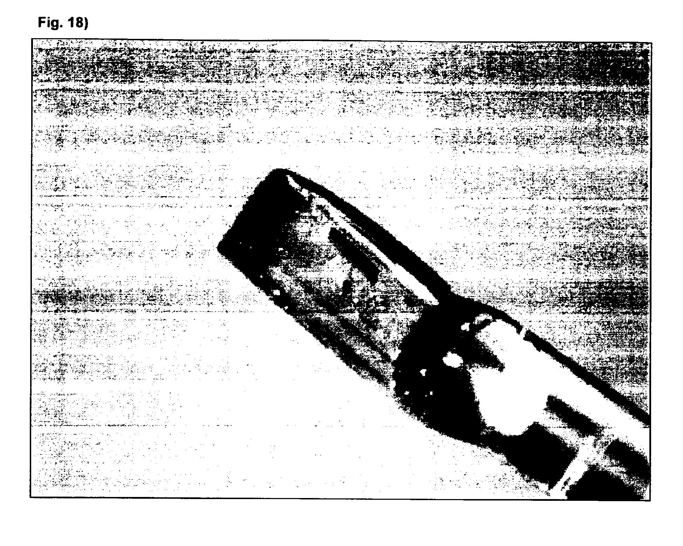

Good quality single crystals were obtained as shown in Fig. 18).

The product has been fully characterized by ¹HNMR, FTIR, X-Ray diffraction, and melting point (see figures 13 to 17).

### FT-IR spectrum (see Fig. 13)

The FTIR spectra of the crystalline form of Memantine-HTB salt are shown measured as described in Example 7.

FTIR spectra were recorded using a Thermo Nicolet Nexus 870 FT-IR, equipped with a beamsplitter KBr system, a 35mW He-Ne laser as the excitation source and a DTGS KBr detector. The spectra were acquired in 32 scans at a resolution of 4 cm⁻¹.

The sample (KBr pellets) shows a Fourier Transform Infra Red spectrum with absorption bands at 3178 (w, br), 2948.9 (m), 2919.2 (m), 2848.8 (m), 1592.5 (s), 1501.2 (m), 1453.7 (m), 1438.1 (s), 1389 (s), 1240 (s), 1175.2 (s), 1152 (m), 1122.4 (s), 921.3 (m) cm⁻¹ (see figure 13).

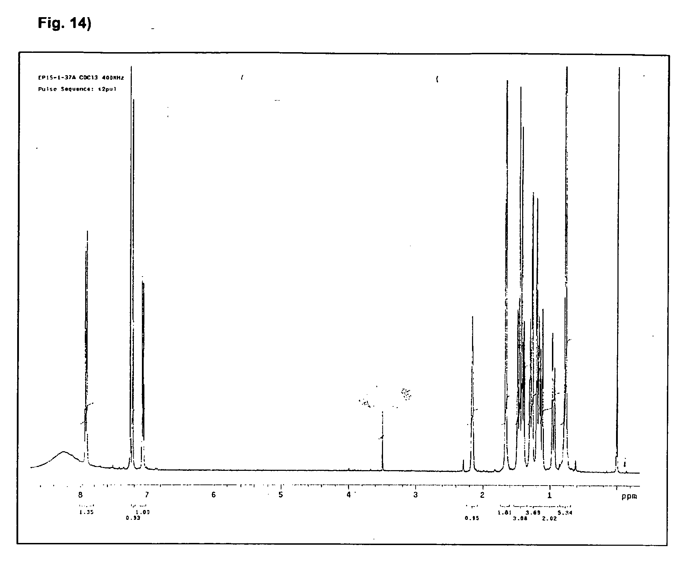

### ¹H-NMR spectrum (see Fig. 14)

Proton nuclear magnetic resonance analyses were recorded in deuterated chloroform (CDCl₃) in a Varian Mercury 400 spectrometer, equipped with a broadband probe ATB 1H/19F/X of 5 mm. Spectra were acquired dissolving 5-10 mg of sample in 0.6 mL of deuterated solvent.

¹H NMR spectrum (see figure 14) in D-chloroform at 400 MHz shows peaks at 7.92 (d, *J* = 8.2 Hz, 1 H) ; 7.22 (s, 1H); 7.07 (d, *J* = 8.2Hz, 1 H) ; 2.19-2.12 (m, 1 H) ; 1.66 (s, 2H) ; 1.47 (d, *J* = 11.5 Hz, 2H ; 1.40 (d, *J* = 11.5 Hz, 2H ) ; 1.29 (d, *J* = 12.6 Hz, 2H) ; 1.19 (d, *J* = 12.6 Hz, 2H) ; 1.13 (d, *J* = 12.7 Hz, 1 H) ; 0.95 (d, *J* = 12.7 Hz, 1 H) ; 0.78 (s, 6H).

### DSC analysis (see Fig. 15)

DSC analyses were recorded with a Mettler DSC822^{e}. A sample of 3.5690 mg was weighed into 40 µL aluminium crucible with a pinhole lid and was heated, under nitrogen (50 mL/min), at 10°C/min from 30 to 300 °C.

The novel type of crystal of the present invention is **characterized in that** the endothermic sharp peak corresponding to the melting point has an onset at 205.73 °C (fusion enthalpy - 67.1 J/g), measured by DSC analysis (10 °C/min) (see figure 15).

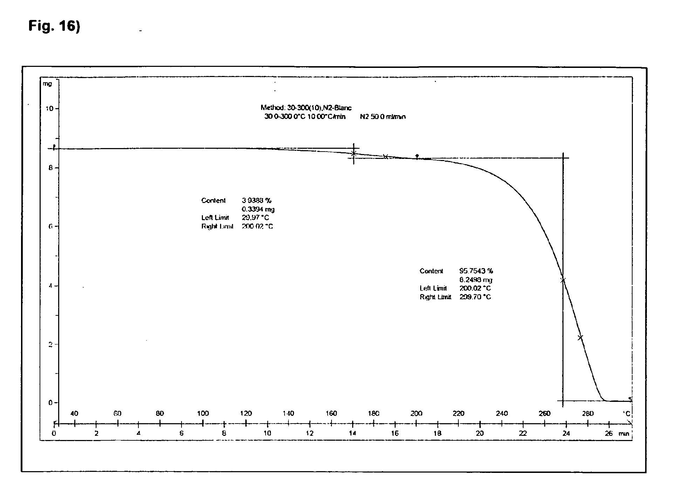

### TG analysis (see Fig. 16)

Thermogravimetric analyses were recorded in a thermogravimetric analyzer Mettler TGA/SDTA851^{e}. A sample of 8.6156 mg was weighed into a 70 µL alumina crucible with a pinhole lid and was heated at 10 °C/min from 30 to 300 °C, under nitrogen (50 mL/min).

The TG analysis of the crystalline form according to the invention shows 3.94% weight loss between 30 and 200 °C corresponding to the presence of impurities derived from the preparation method (no purification) (see figure 16).

### Powder X-Ray diffraction pattern (see Fig. 17A)

XRPD analysis was performed using a Philips X'Pert diffractometer with Cu K_{α} radiation in Bragg-Brentano geometry. The system is equipped with a proportional detector. The measurement parameters were as follows: the range of 2θ was 3° to 40° at a scan rate of 1.8° per minute (see figure 17A).

List of selected peaks:

| **2θ (°)** | **d (Å)** | **I (%)** |
|---|---|---|
| 6.71 | 13.18 | 26 |
| 8.57 | 10.32 | 53 |
| 10.23 | 8.65 | 4 |
| 11.39 | 7.77 | 6 |
| 13.31 | 6.65 | 100 |
| 14.15 | 6.26 | 6 |
| 14.98 | 5.91 | 34 |
| 15.51 | 5.72 | 25 |
| 16.56 | 5.35 | 14 |
| 17.07 | 5.20 | 3 |
| 17.61 | 5.04 | 25 |
| 17.90 | 4.96 | 14 |
| 18.34 | 4.84 | 15 |
| 18.95 | 4.68 | 35 |
| 19.84 | 4.48 | 2 |
| 20.82 | 4.27 | 7 |
| 22.10 | 4.02 | 5 |
| 22.54 | 3.94 | 3 |
| 22.76 | 3.91 | 3 |
| 24.50 | 3.63 | 2 |
| 25.30 | 3.52 | 7 |
| 25.76 | 3.46 | 5 |
| 26.68 | 3.34 | 3 |
| 27.15 | 3.28 | 2 |
| 29.29 | 3.05 | 2 |
| 32.80 | 2.73 | 2 |
| 39.46 | 2.28 | 1 |

In addition the Powder X-Ray diffraction pattern of the starting products memantine base and HTBwere compared to the XRPD above (Fig. 17B), proving the formation of the salt.

### Example 6: Single Crystal XRD analysis of a single crystal of memantine-HTB salt.

The crystal structure of the memantine-HTB salt has been determined from single crystal X-ray diffraction data. The colourless prism (0.56 × 0.33 × 0.12 mm) used was obtained from the cold evaporation of a methanol solution of equimolar amounts of Memantine and HTB.

Analysis was performed at room temperature using a Bruker Smart Apex diffractometer with graphite monochromated Mo Kα radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0). The structure was solved with direct methods and least-squares refinement of Fo2 against all measured intensities was carried out (program used: SHELXTL -NT 6.1). All non-hydrogen atoms were refined with anisotropic displacement parameters.

Relevant structural data:

| Crystal system | Triclinic |
|---|---|
| Space group | P-1 |
| a (Å) | 7.1296(6) |
| b (Å) | 11.0891(9) |
| c (Å) | 13.5470(12) |
| α (°) | 94.453(2) |
| β (°) | 94.769(2) |
| γ (°) | 108.368(2) |
| Volume (Å3) | 1006.83(15) |
| Z | 2 |
| D calc. (Mg/m3) | 1.271 |
| N. of refl. | 4700 |
| Refl. with I > 2σ(I) | 3058 |
| R (I > 2σ(I)) | 0.0698 |

Refinement on *P***1** space group leads to an R index of 5.6%.

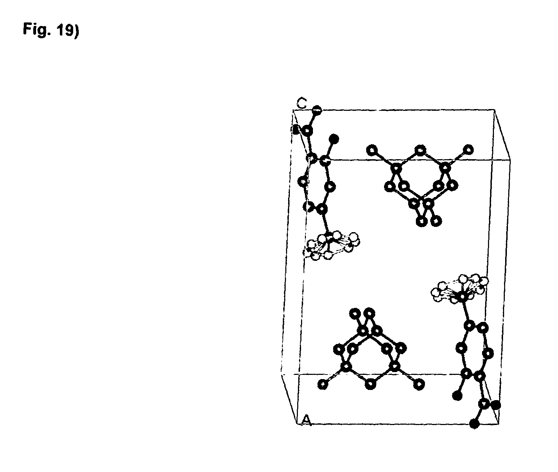

The crystal structure is depicted in figure 19.

Simulation of the XRPD diffractogram from single crystal data gives an almost identical diagram to the experimental one presented above. From these single crystals powder diffraction patterns were acquired. The positions of the peaks coincided with those in Figure 17 indicating that the product obtained according to example 5 only gives one crystalline phase.

## Claims

1. A salt of Memantine with a COX-INHIBITOR, **characterized in that** the COX-INHIBITOR has a carboxylic group.

2. The salt according to claim 1, **characterized in that** the COX-INHIBITOR is selected from:
- Acetylsalicylic Acid;
- Triflusal;
- HTB (2-hydroxy-4-trifluoromethyl benzoic acid);
- Diflunisal;
- Meclofenamic acid;
- Mefenamic acid;
- Niflumic acid;
- Flufenamic acid.
- Diclofenac;
- Lonazolac;
- Acemetacin;
- Indomethacin;
- Tolmetin;
- Sulindac
- Etodolac;
- Keterolac
- Flurbiprofen;
- (RS)-Flurbiprofen;
- Esflurbiprofen;
- Ibuprofen;
- (RS)-Ibuprofen;
- S-(+)-Ibuprofen;
- Ketoprofen;
- (rac)-Ketoprofen
- R-(-)-Ketoprofen
- Bermoprofen;
- Pelubiprofen;
- Tenosal;
- Aceneuramic acid;
- Pirazolac;
- Xinoprofen;
- Flobufen;
- Anirolac;
- Zoliprofen;
- Bromfenac;
- Pemedolac;
- Dexpemedolac;
- Bindarit;
- Romazarit;
- Naproxen;
- (S)-Naproxen;
- Tiaprofenic acid;
- Ketorolac;
- Fenbufen;
- Fenoprofen;
- Flobufen; or
- Oxaprozin.

3. The salt according to claim 1, **characterized in that** the COX-INHIBITOR is selected from:
• Salicylates,
• Anthranilates,
• Arylacetic Acids/ Arylalkanoic Acids,
• Arylpropionic Acids.

4. The salt according to claim 3, **characterized in that** the Salicylates are selected from:
- Acetylsalicylic Acid;
- Triflusal;
- HTB (2-hydroxy-4-trifluoromethyl benzoic acid); or
- Diflunisal;
preferably is
- Acetyl salicylic acid;
- HTB; or
- Triflusal.

5. The salt according to claim 3, **characterized in that** the Anthranilates are selected from:
- Meclofenamic acid;
- Mefenamic acid;
- Niflumic acid; or
- Flufenamic acid.

6. The salt according to claim 3, **characterized in that** the Arylacetic Acids/Arylalkanoic Acids are selected from:
- Diclofenac;
- Lonazolac;
- Acemetacin;
- Indomethacin;
- Tolmetin; or
- Sulindac
- Etodolac;
- Keterolac
, preferably from
- Diclofenac;
- Lonazolac;
- Acemetacin;
- Indomethacin;
- Tolmetin; or
- Sulindac
, most preferably is
- Diclofenac.

7. The salt according to claim 3, **characterized in that** the Arylpropionic Acids are selected from:
- Flurbiprofen;
- (RS)-Flurbiprofen;
- Esflurbiprofen;
- Ibuprofen;
- (RS)-Ibuprofen;
- S-(+)-Ibuprofen;
- Ketoprofen;
- (rac)-Ketoprofen;
- R-(-)-Ketoprofen;
- Naproxen;
- (S)-Naproxen;
- Tiaprofenic acid;
- Ketorolac;
- Fenbufen;
- Fenoprofen;
- Flobufen;
- Oxaprozin;
- Tolmetin;
- Xinoprofen;
- Flobufen;
- Zoliprofen;
- Bermoprofen; or
- Pelubiprofen;
, preferably from
- Flurbiprofen;
- (RS)-Flurbiprofen;
- Esflurbiprofen;
- Ibuprofen;
- (RS)-Ibuprofen;
- S-(+)-Ibuprofen;
- Ketoprofen;
- (rac)-Ketoprofen;
- R-(-)-Ketoprofen;
- Naproxen;
- (S)-Naproxen;
- Tiaprofenic acid; or
- Ketorolac;
preferably is
- (RS)-Flurbiprofen;
- Esflurbiprofen;
- (RS)-Ibuprofen;
- S-(+)-Ibuprofen;
- (rac)-Ketoprofen;
- R-(-)-Ketoprofen; or
- (S)-Naproxen.

8. A salt of Memantine with a COX-INHIBITOR according to claim 1 selected from Memantine-Ibuprofen salt, Memantine-Flurbiprofen salt, Memantine-Diclofenac salt, Memantine-Acetylsalicylic Acid salt, Memantine-(S)-Naproxen salt, Memantine/Triflusal salt, or Memantine/2-hydroxy-4-trifluoromethyl benzoic acid (HTB) salt.

9. Crystalline form of a salt according to any of claims 1 to 8.

10. Process for the production of a salt according to claim 1 comprising the steps of:
dissolving a COX-INHIBITOR with a carboxylic group either as a free acid or as a salt together with, or after, or before, memantine either as a free base or as a salt in an organic solvent,
stirring the mixture obtained at a temperature between 0°C and 80°C, filtering the obtained solid and/or evaporating the solvent, and drying of the resulting product.

11. Process according to claim 10, wherein
• the organic solvent is selected from acetone, acetonitrile, isobutyl acetate, hepatane, methanol, tetrahydrofuran, isopropanol, ethanol or cyclohexane; and/or
• the solvent is evaporated under high vacuum; and/or
• the ratio of memantine to COX-INHIBITOR is 1:1 to 2:1, preferably 1:1; and/or
• the memantine dissolved is a free base.

12. Medicament comprising at least one salt according to any of claims 1 to 8 and optionally one or more pharmaceutically acceptable excipients.

13. Pharmaceutical composition **characterized in that** it comprises a therapeutically effective amount of the crystalline form of a salt according to any one of claims 1 to 8, in a physiologically acceptable medium.

14. Use of a salt according to any one of claims 1 to 8 for the treatment of pain, preferably acute pain, chronic pain, neuropathic pain, hyperalgesia, allodynia or cancer pain, including diabetic neuropathy and osteoarthritis.

15. Crystalline form of a Memantine-(S)-Naproxen salt according to claim 9.

16. Crystalline form according to claim 15, **characterized in that** it shows a Fourier Transform Infra Red pattern with absorption bands at 2947, 2906, 2864, 2848, 2648, 2555, 2195, 1633, 1604, 1553, 1536, 1378, 1361, 1346, 1247, 1211, 1036, 858, 814, and 693 cm⁻¹.

17. Crystalline form according to claim 15, **characterized in that** it crystallizes as a monoclinic unit with the following dimensions :
• a = 24.34 Å
• b = 6.65 Å
• c = 15.63 Å
• β angle of 109.18°

18. Crystalline form according to claim 15, **characterized in that** the endothermic sharp peak corresponding to the melting point has an onset at 173 °C.

19. Crystalline form of a Memantine/Triflusal salt according to claim 9.

20. Crystalline form according to claim 19, **characterized in that** it shows a Fourier Transform Infra Red pattern with absorption bands at 2947, 2908, 2867, 1777, 1629, 1587, 1560, 1411, 1385, 1366, 1333, 1207, 1196, 1170, 1159, 1128, 1108, 1065, 944, and 897 cm⁻¹.

21. Crystalline form according to claim 19, **characterized in that** it crystallizes as a monoclinic unit cell with the following dimensions :
• a = 30.96 Å
• b = 13.62 Å
• c = 11.94 Å
• β angle of 112.34°.

22. Crystalline form according to claim 19, **characterized in that** the endothermic sharp peak corresponding to the melting point has an onset at 133 °C.

23. Crystalline form of a Memantine/HTB salt according to claim 9.

24. Crystalline form according to claim 23, **characterized in that** it shows a Fourier Transform Infra Red pattern with absorption bands at 2949, 2919, 2849, 1668, 1593, 1501, 1454, 1438, 1389, 1354, 1336, 1256, 1240, 1175, 1152, 1122, 1062, 921, 972, 846, 828, 797, 750, 703, 578 and 490 cm⁻¹.

25. Crystalline form according to claim 23, **characterized in that** it crystallizes as a triclinic unit cell with the following dimensions :
• a = 7.13 Å
• b = 11.09 Å
• c = 13.55 Å
• α angle of 94.45°
• β angle of 94.77°
• γ angle of 108.37°.

26. Crystalline form according to claim 23, **characterized in that** the endothermic sharp peak corresponding to the melting point has an onset at 206 °C.
